# FASCICULE DE BREVET EUROPEEN

(11) **EP 0 982 310 B1**
(45) Date de publication et mention de la délivrance du brevet: **31.03.2004**
(21) Numéro de dépôt: 99114398.3
(22) Date de dépôt: 22.07.1999
(51) Int. Cl.: C07F 7/08, C07F 7/21, A61K 7/42

(54) **Procédé de photostabilisation de filtres solaires dérivés du dibenzoylméthane, compositions cosmétiques filtrantes photostabilisées ainsi obtenues et leurs utilisations.**
Verfahren zur Photostabilisierung von Sonnenschutzfiltern auf Dibenzoylmethanbasis, erhaltene photostabilisierte filternde kosmetische Zusammensetzungen und ihre Verwendungen
Process for photostabilizing dibenzoylmethane-based sunscreens , cosmetic photostabilized sunscreen compositions obtained thereby, and their use

(30) Priorité: 21.08.1998 FR 9810632
(43) Date de publication de la demande: 01.03.2000
(73) Titulaire: L'OREAL, 75008 Paris (FR)
(72) Inventeur: Forestier, Serge, 77410 Claye-Souilly (FR); Richard, Hervé, 93420 Villepinte (FR)
(74) Mandataire: Casalonga, Axel

(56) Documents cités:
- EP-A- 0 675 108
- EP-A- 0 709 080
- WO-A-93/10745
- CHEMICAL ABSTRACTS, vol. 119, no. 24, 13 décembre 1993 (1993-12-13) Columbus, Ohio, US; abstract no. 251293, FRIEDRICH, HOLGER ET AL: "Polymeric light stabilizers based on siloxanes" XP002098917 & POLYM. DEGRAD. STAB. (1993), 42(2), 127-44 CODEN: PDSTDW;ISSN: 0141-3910,1993,
- CHEMICAL ABSTRACTS, vol. 118, no. 8, 22 février 1993 (1993-02-22) Columbus, Ohio, US; abstract no. 66597, OKAZAKI, TOMOMI ET AL: "Cosmetics containing benzophenone silicones as UV absorbers" XP002098918 & JP 04 217622 A (SHISEIDO CO., LTD., JAPAN)
- CHEMICAL ABSTRACTS, vol. 114, no. 20, 20 mai 1991 (1991-05-20) Columbus, Ohio, US; abstract no. 186892, YOSHIKAWA, KAZUMI ET AL: "Polymer compositions containing hydroxybenzophenone derivatives for light resistance" XP002098919 & JP 02 187437 A (ADEKA ARGUS CHEMICAL CO., LTD., JAPAN)
- CHEMICAL ABSTRACTS, vol. 110, no. 24, 12 juin 1989 (1989-06-12) Columbus, Ohio, US; abstract no. 213551, JANSEN, I. ET AL: "Synthesis of styrene- and butadiene-cyclosiloxane block copolymers containing UV-absorber groups" XP002098920 & ACTA POLYM. (1989), 40(2), 116-21 CODEN: ACPODY;ISSN: 0323-7648,1989,
- CHEMICAL ABSTRACTS, vol. 116, no. 21, 25 mai 1992 (1992-05-25) Columbus, Ohio, US; abstract no. 214698, YOKOGAWA, YOSHIHIRO ET AL: "Preparation of silyl-substituted benzophenone compound as UV-absorbing skin protectant" XP002098921 & JP 03 287589 A (SHISEIDO CO., LTD., JAPAN)
- CHEMICAL ABSTRACTS, vol. 116, no. 19, 11 mai 1992 (1992-05-11) Columbus, Ohio, US; abstract no. 194587, YOSHIDA, MASASHI ET AL: "Preparation of silyl-substituted benzophenone derivatives as UV-absorbing skin protectant" XP002098922 & JP 03 287588 A (SHISEIDO CO., LTD., JAPAN)

## Description

La présenté invention se rapporte à un nouveau procédé pour améliorer la stabilité d'au moins un dérivé du dibenzoylméthane vis-à-vis du rayonnement UV consistant à associer au dit dérivé de dibenzoylméthane une quantité efficace d'un composé organosiloxanique à fonction 2-hydroxybenzophénone.

On sait que les radiations lumineuses de longueurs d'onde comprises entre 280 nm et 400 nm permettent le brunissement de l'épiderme humain, et que les rayons de longueurs d'onde plus particulièrement comprises entre 280.et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré: Ils favorisent le déclenchement de la réaction érythémateuse ou amplifient cette réaction chez certains sujets et peuvent même être à l'origine de réactions phototoxiques ou. photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'absorption intrinsèque. Ces dérivés du dibenzoylméthane, qui sont maintenant des produits bien connus en soi à titre de filtres actifs dans l'UV-A, sont notamment décrits dans les demandes de brevets français FR-A-2326405 et FR-A-2440933, ainsi que dans la demande de brevet européen EP-A-0114607 ; le 4-(ter.-butyl) 4'-méthoxy dibenzoyl-méthane est par ailleurs actuellement proposé à la vente sous la dénomination commerciale de " ®PARSOL 1789" par la Société GIVAUDAN.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que, de façon contraignante, des applications répétées à intervalles de temps réguliers et rapprochés doivent être effectuées par l'utilisateur pour obtenir une protection efficace de la peau contre les rayons UV.

Ainsi, il a été proposé dans le document EP-A-0709080 d'associer aux dérivés du dibenzoylméthane des dérivés de benzalmalonate afin de diminuer la photoinstabilité desdits dérivés de dibenzoylméthane. Toutefois, la photostabilisation des dérivés du dibenzoylméthane vis-à-vis du rayonnement UV constitue, à ce jour, un problème qui n'a pas encore été résolu de manière complètement satisfaisante.

Il a été également proposé dans le document EP-A-0843996 d'associer aux dérivés du dibenzoylméthane des dérivés hydrocarbonés du 2-hydroxybenzophénone tels que la 2-hydroxy-4-méthoxy benzophénone ou l'acide 2-hydroxy-4-méthoxybenzophénone-5-sulfonique afin de diminuer la photo-instabilité desdits dérivés de dibenzoylméthane.

Une autre difficulté, indépendante de celle évoquée ci-avant, rencontrée avec les dérivés de dibenzoylméthane est qu'il s'agit de filtres lipophiles présentant la particularité mais aussi le désavantage d'être solides à température ambiante. De ce fait, leur utilisation dans une composition cosmétique antisolaire implique certaines contraintes au niveau de leur formulation et de leur mise en oeuvre, en particulier lorsqu'il s'agit de trouver des solvants permettant de les solubiliser correctement, seuls ou conjointement avec d'autres filtres. A cet égard, on fait aujourd'hui le plus souvent appel à des huiles comme des esters et plus particulièrement des benzoates d'alkyles en C₁₂-C₁₅ (" ®FINSOLV TN" de chez Finetex), ou à des triglycérides et notamment des triglycérides d'acides gras en C₈-C₁₂ ("®MIGLYOL 812" de chez Hüls), mais ces différents produits possèdent des propriétés solubilisantes vis-à-vis des filtres susmentionnés qui peuvent apparaître comme encore insuffisantes.

Les formulations antisolaires à base de dérivés de dibenzoylméthane et du 2-hydroxy-4-méthoxybenzophénone ou de l'acide 2-hydroxy-4-méthoxybenzophénone-5-sulfonique telles que mises en oeuvre dans le document EP-A-0843996, ne permettent pas de manière satisfaisante de résoudre ce problème de solubilité desdits dérivés de dibenzoylméthane.

On a déjà proposé dans la demande de brevet japonais JP-A-04217622 et dans la demande de brevet WO93/10745 d'utiliser comme filtres UV dans des formulations photoprotectrices pour la peau des composés organopolysiloxanes à fonction 2-hydroxybenzophénone.

On sait également dans la demande de brevet japonais JP-A-02187437, la demande de brevet EP-A-0675 108, les articles CA vol. 119, N° 24 résumé 251293 et CA vol. 110 N°24 résumé N°213551 que certains composés organosiloxanes à fonction hydroxybenzophénone pouvaient être utilisés dans la synthèse de polymères spécifiques comme photostabilisants.

Or, la Demanderesse vient maintenant de découvrir, de façon surprenante, qu'en associant aux dérivés du dibenzoylméthane mentionnés ci-dessus une quantité efficace d'un composé organosiloxanique à fonction 2-hydroxybenzophénone, il était possible d'améliorer de manière substantielle et remarquable, la stabilité photochimique (ou photostabilité) de ces mêmes dérivés du dibenzoylméthane.

Il a été également trouvé, et il s'agit là de l'un des avantages supplémentaires attachés à la présente invention, que certains composés organosiloxaniques à fonction 2-hydroxybenzophénone utilisables dans le cadre de la présente invention à titre d'agents photostabilisants constituent par ailleurs, de façon également très surprenante par rapport aux filtres organiques hydrocarbonés dérivés de 2-hydroxybenzophénone connus, des solvants particulièrement remarquables pour les filtres du type dérivés du dibenzoylméthane comme par exemple le 4-(ter.-butyl)-4'-méthoxy dibenzoylméthane ; ce qui permet, à quantité égale de solvant, de mettre en oeuvre des quantités plus importantes de filtres UV.

Ainsi, conformément à un premier objet de la présente invention, il est proposé un nouveau procédé pour améliorer la stabilité d'au moins un dérivé du dibenzoylméthane vis-à-vis du rayonnement UV, consistant à associer au dit dérivé du dibenzoylméthane une quantité efficace d'un dérivé organosiloxanique à fonction 2-hydroxybenzophénone.

Les compositions cosmétiques et/ou dermatologiques obtenues conformément au procédé de l'invention présentent l'avantage d'être particulièrement photostables, même après une exposition prolongée aux rayonnements UV-A et UV-B. Ces rayonnements peuvent être d'origine naturelle (soleil) ou artificielle (lampe UV).

La présente invention a également pour objet l'utilisation d'un composé organosiloxanique à fonction 2-hydroxybenzophénone dans la préparation d'une composition cosmétique ou dermatologique contenant au moins un dérivé du dibenzoyl-méthane, pour améliorer la stabilité dudit dérivé du dibenzoylméthane vis-à-vis du rayonnement UV.

D'autres caractéristiques, aspects et avantages de la présente invention apparaîtront à la lecture de la description détaillée qui va suivre.

. Comme indiqué précédemment, les dérivés du dibenzoylméthane destinés à être photostabilisés selon la présente invention sont des produits déjà bien connus en soi et décrits notamment dans les documents FR-A-2326405, FR-A-2440933 et EP-A-0114607 précités.

Selon la présente invention, on peut bien entendu mettre en oeuvre un ou plusieurs dérivés du dibenzoylméthane.

Parmi les dérivés du dibenzoylméthane utilisables selon la présente invention; on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,

Parmi les dérivés du dibenzoylméthane mentionnés ci-dessus, on préfère tout particulièrement, selon la présente invention, mettre en oeuvre le 4-(tert.-butyl) 4'-méthoxy dibenzoylméthane, notamment celui proposé à la vente sous la dénomination commerciale de " ®PARSOL 1789" par la Société GIVAUDAN, ce filtre répondant donc à la formule développée suivante :

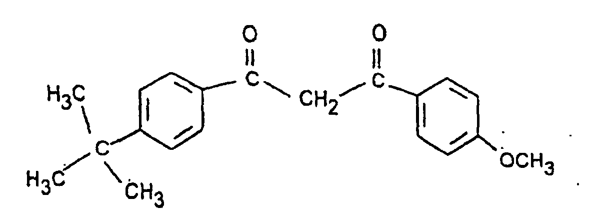

Un autre dérivé du dibenzoylméthane préféré selon la présente invention est le 4-isopropyl-dibenzoylméthane, filtre vendu sous la dénomination de " ®EUSOLEX 8020" par la Société MERCK, et répondant à la formule développée suivante :

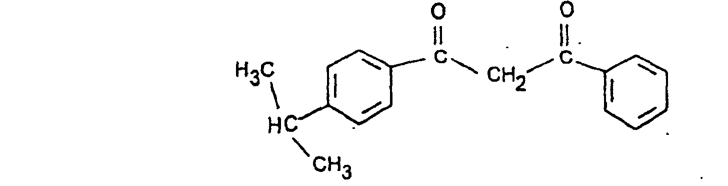

Le ou les dérivés du dibenzoylméthane peuvent être présents dans les compositions destinées à être stabilisées conformément au procédé de l'invention, à des teneurs allant généralement de 0,01 % à 10 % en poids, et de préférence à des teneurs allant de 0,3 % à 5 % en poids, par rapport au poids total de la composition.

Le composé organosiloxane à groupement 2-hydroxybenzophénone utilisé dans la présente invention répond aux formules suivantes:

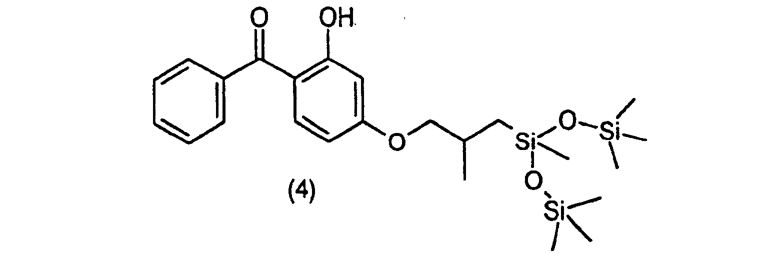

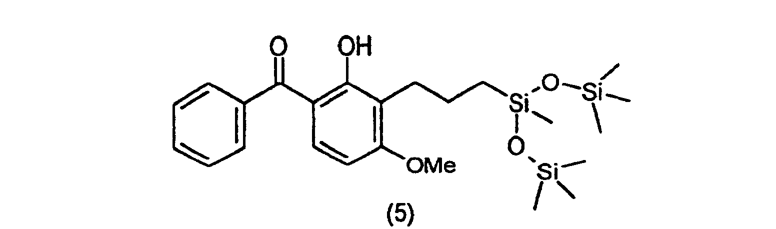

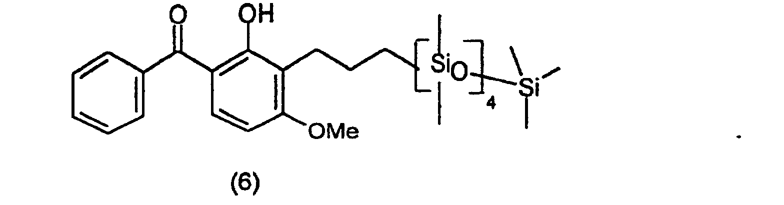

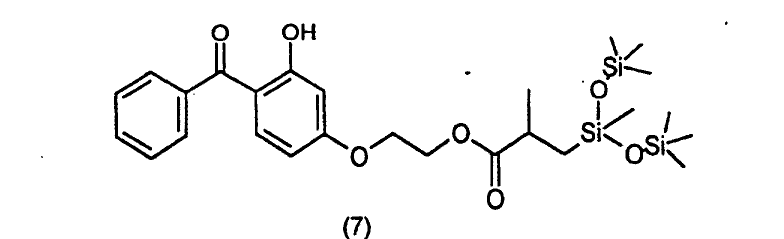

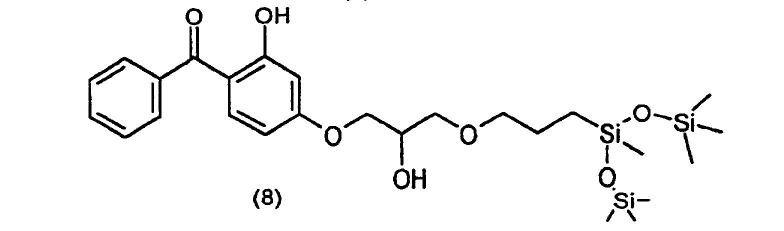

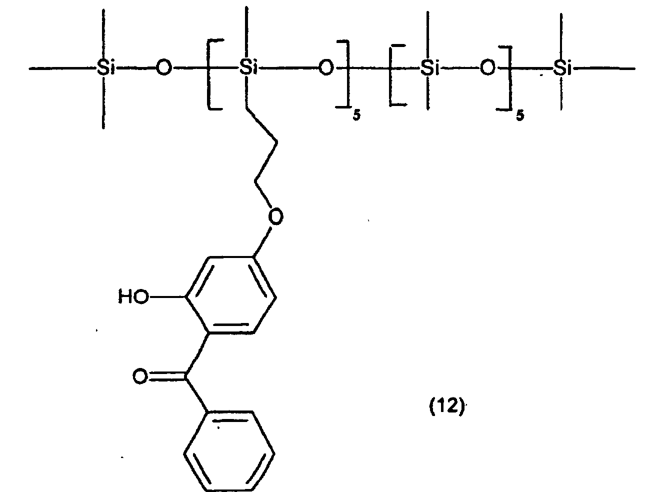

Ces composés, ainsi que leur procédé de préparation, sont décrits en particulier dans les documents FR-A-2657351 et EP-A-0655453 et EP-A - 0389337.

Parmi les composés tels que définis ci-dessus, certains d'entre eux sont nouveaux et constituent un autre objet de la présente invention.

Parmi ces composés particuliers, on peut citer le composé (8) tel que décrit précédemment.

Par quantité efficace de composé organosiloxane à fonction ,2-hydroxybenzophénone selon l'invention, on entend une quantité suffisante pour obtenir une amélioration notable et significative de la photostabilité du ou des dérivés du dibenzoylméthane contenus dans la composition. La quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que décrit dans la demande FR-A-2607700.

Les composés organosiloxaniques à fonction 2-hydroxybenzophénone sont généralement présents dans les compositions selon l'invention à une teneur au moins égale à 0,5 % en poids, par rapport au poids total de la composition. De préférence encore, cette teneur va de 0,5 % à 20 % en poids, par rapport au poids total de la composition.

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles. Ces filtres complémentaires peuvent être notamment choisis parmi les dérivés cinnamiques, les dérivés salicyliques, les dérivés du benzylidène camphre, les dérivés de benzotriazole, les dérivés de benzimidazole, les dérivés de triazine, les dérivés du benzalmalonate, les dérivés de β,β'-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A-0487404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A-0518773.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents antiradicaux libres, les opacifiants, les stabilisants, les émollients, les silicones, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions. Bien entendu, tous les ingrédients supplémentaires susceptibles d'être introduits dans les compositions conformes à l'invention doivent être tels qu'ils ne perturbent ou n'altèrent substantiellement pas l'effet de photostabilisation exercé par les composés organosiloxaniques à fonction 2-hydroxybenzophénone sur les dérivés du dibenzoylméthane.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale «®Finsolv TN» par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR-A-2315991 et FR-A-2416008).

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, et peut constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

Des exemples concrets illustrant l'invention vont maintenant être donnés.

### EXEMPLE 1 :

### Préparation du dérivé de formule (8) :

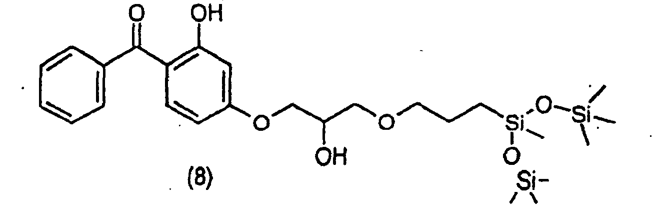

On porte à 110 °C sous barbotage d'azote pendant 8 heures, un mélange de 2,4-dihydroxy benzophénone (10,7 g, 0,05 mole) et de 3-glycidyloxypropyl-Bis-(triméthyl-siloxy)-méthyl silane (17,2 g, 0,051 mole) en présence d'iodure de tétrabutyl ammonium (0,2 g). L' huile brute obtenue a été chromatographiée sur Silice (éluant : dichlorométhane/heptane 60:40 puis gradient jusque dichlorométhane seul) pour donner en fractions milieux 14,7 g (Rendement : 53 %) du dérivé de l'exemple 1 sous forme d'une huile visqueuse jaune pâle :
- UV (Ethanol) λₘₐₓ = 286 nm, εₘₐₓ = 15 090
   λₘₐₓ = 326 nm, εₘₐₓ = 9 890

### EXEMPLE 2 :

### Préparation du dérivé de formule (4) :

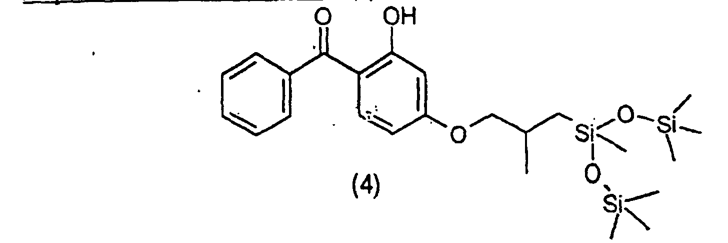

On porte à 90°C sous agitation et barbotage d'azote un mélange de 2,4-hydroxy benzophénone (2,14 g, 0,01 mole), de carbonate de potassium (2,76 g) et d'iodure de potassium (0,276 g) dans 20 ml de DMF. On ajoute en 15 minutes à 90°C le chloroisobutyl heptamethyl trisiloxane (6,26 g, 0,02 mole). On chauffe à 90-100°C pendant 16 heures. On verse le mélange réactionnel dans la glace et l'extrait au dichloro méthane. La phase organique est récupérée, lavée à l'eau, séchée sur sulfate de sodium et concentrée. L' huile brute obtenue a été chromatographiée sur Silice (éluant : dichlorométhane/heptane 50:50) pour donner 2,1 g (Rendement : 43 %) du dérivé de l'exemple 3 sous forme d'une huile jaune pâle :
- UV (Ethanol) λₘₐₓ = 286 nm, εₘₐₓ = 15 550 λₘₐₓ = 326 nm, εₘₐₓ = 9 920

### EXEMPLE 3 :

On a réalisé les compositions A et B suivantes (les quantités sont exprimées en poids % par rapport au poids total de la composition) :

| **Ingrédients** | **Composition A (comparative)** | **Composition B (invention)** |
|---|---|---|
| Myristate d'isopropyle | 30 | 30 |
| Parsol 1789 | 1,5 | 1,5 |
| composé de formule (5) | - | 5 |
| Ethanol | qsp 100 | qsp 100 |

La composition B (invention) indiquée ci-dessus comprend en outre 5% en poids d'un composé (5) siliconé à fonction 2-hydroxybenzophénone selon l'invention répondant à la formule (I) telle que définie ci-dessus et ayant pour structure:

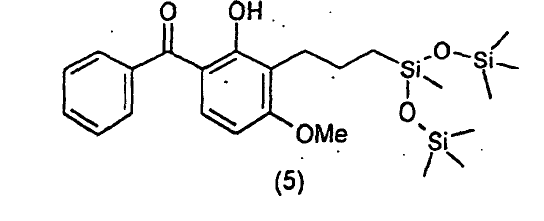

Ce composé est décrit dans le brevet FR-A-2657351 et synthétisé selon le mode préparatoire indiqué dans l'exemple 1 dudit brevet.

Pour chacune de ces compositions, on a déterminé le pourcentage de Parsol 1789 (4-tert-butyl-4'-méthoxydibenzoylméthane) résiduel après irradiation par des UV selon le protocole suivant :

Pour chaque formule on a préparé 3 échantillons tests et 3 échantillons témoins. On a déposé à la micropipette 2 µl/cm² de formule sur des plaques de Quartz dépoli. Les plaques tests ont été exposées 2h50mn au Suntest HERAEUS muni d'une lampe XENON 1kW ( ce qui correspond à un équivalent de 1.7 heures UVA et 3 heures UVB) et les plaques témoins sont conservées pendant le même temps et à la même température (35-40°C) à l'obscurité. A l'issue de ce temps, on a procédé à l'extraction des filtres en immergeant chaque plaque dans 50g d'éthanol absolu, et en les passant aux ultrasons pendant 15 mn pour assurer une bonne extraction. Les solutions obtenues sont analysées par chromatographie en phase liquide haute performance. Pour chaque formule testée, le taux de Parsol 1789 résiduel après exposition est donné par le rapport de sa concentration dans l'échantillon exposé à sa concentration dans l'échantillon non exposé.

Les résultats obtenus sont rassemblés dans le tableau ci-dessous :

| **COMPOSITION** | **PARSOL 1789 RESIDUEL** |
|---|---|
| **A (comparative)** | 2% |
| **B (invention)** | 36% |

Ces résultats montrent que la présence du composé siliconé (5) améliore la photostabilité du PARSOL 1789 après exposition aux UV.

## Revendications

1. Procédé pour améliorer la stabilité d'au moins un dérivé du dibenzoylméthane vis-à-vis du rayonnement UV, **caractérisé par le fait qu'**il consiste à associer audit dérivé du dibenzoylméthane une quantité efficace d'un composé organosiloxanique à fonction 2-hydroxybenzophénone, choisi parmi les composés de formules suivantes :

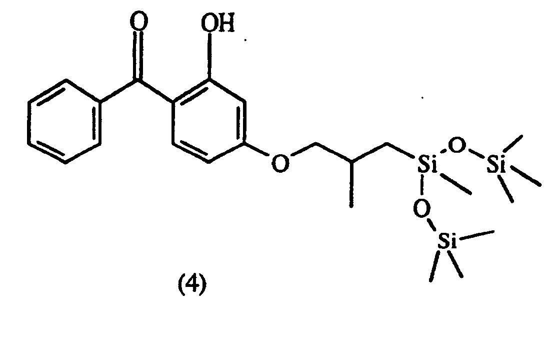

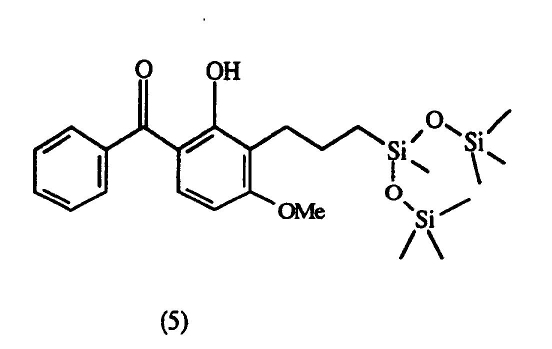

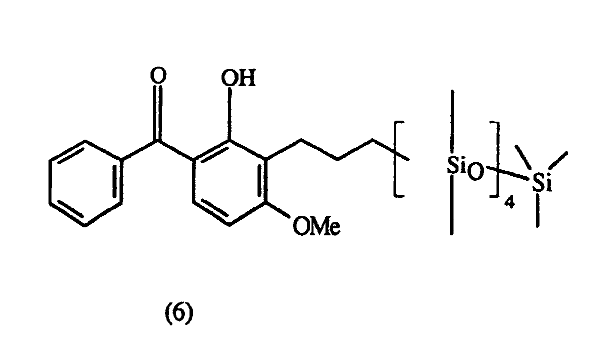

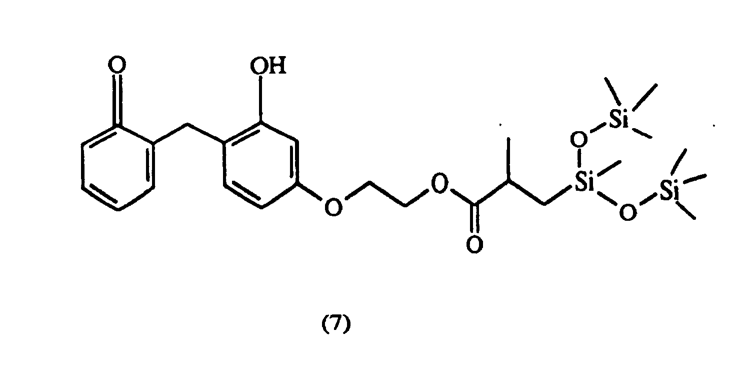

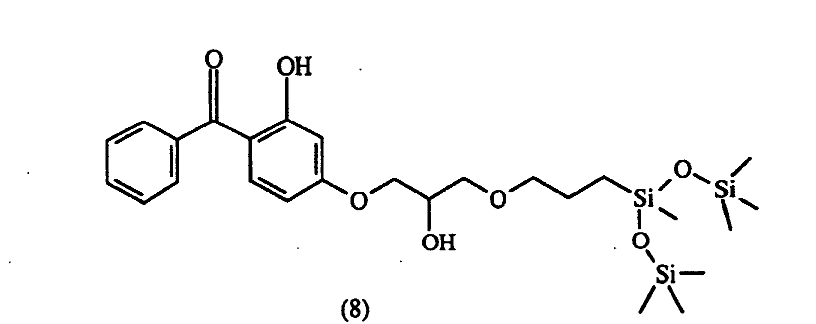

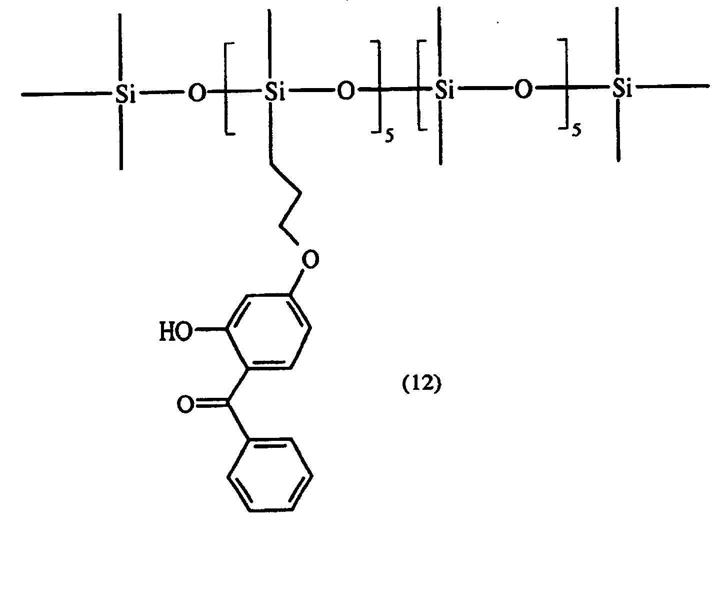

2. Procédé selon la revendication 1 où le composé organosiloxanique est le composé de structure :

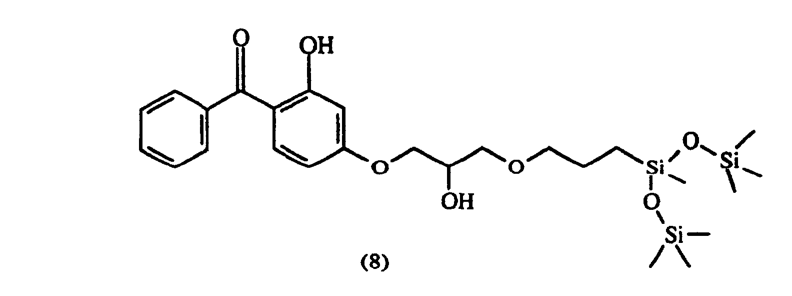

3. Procédé selon l'une quelconque des revendications 1 ou 2, **caractérisé par le fait que** le dérivé de dibenzoylméthane est choisi parmi :
- le 2-méthyldibenzoylméthane
- le 4-méthyldibenzoylméthane
- le 4-isopropyldilbenzoylméthane
- le 4 tert.-butyldibenzoylméthane
- le 2,4-diméthyldibenzoylméthane
- le 2,5 diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

4. Procédé selon la revendication 3, **caractérisé par le fait que** le dérivé de dibenzoylméthane est le 4-(tert.-butyl) 4'-méthoxydibenzoylméthane.

5. Procédé selon la revendication 3, **caractérisé par le fait que** le dérivé de dibenzoylméthane est le 4-isopropyl-dibenzoylméthane.

6. Composition cosmétique filtrante photostable pour la photoprotection par voie topique de la peau et/ou des cheveux contre le rayonnement ultraviolet, en particulier le rayonnement solaire, du type comprenant, dans un support cosmétiquement acceptable, au moins un dérivé du dibenzoylméthane, **caractérisée par le fait qu'**elle comprend en outre une quantité efficace d'au moins un composé organosiloxanique à fonction 2-hydroxybenzophénone choisi parmi les composés de formules suivantes :

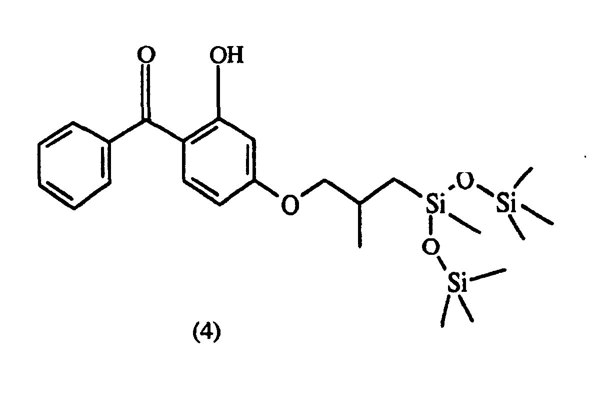

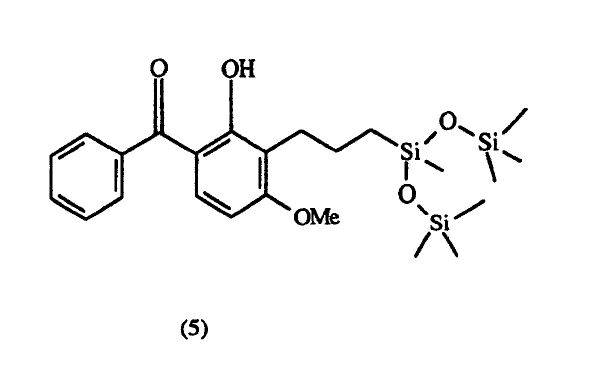

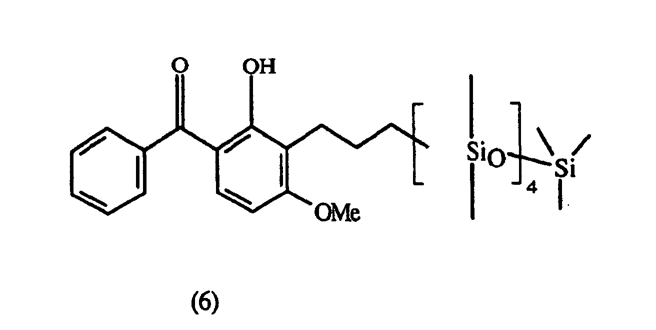

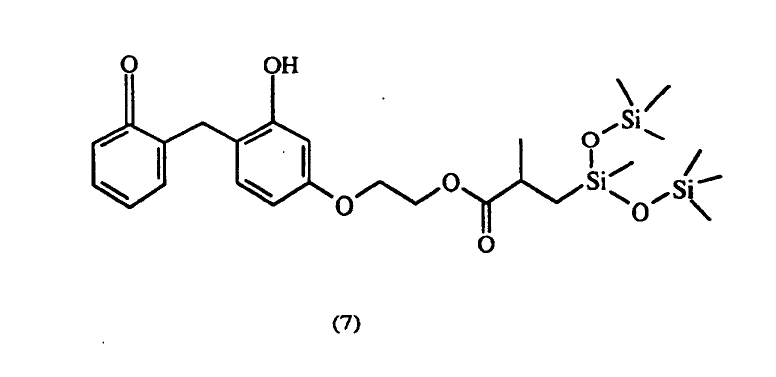

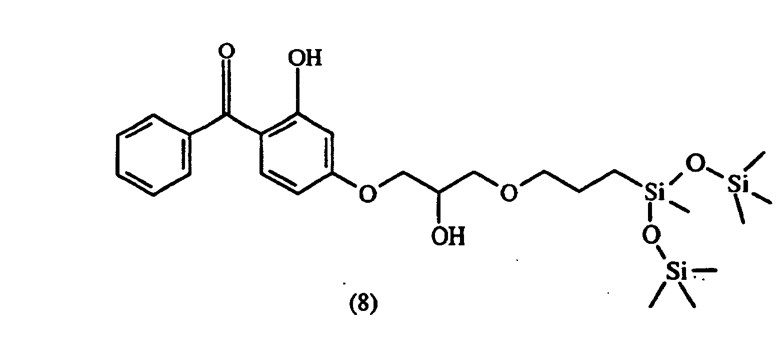

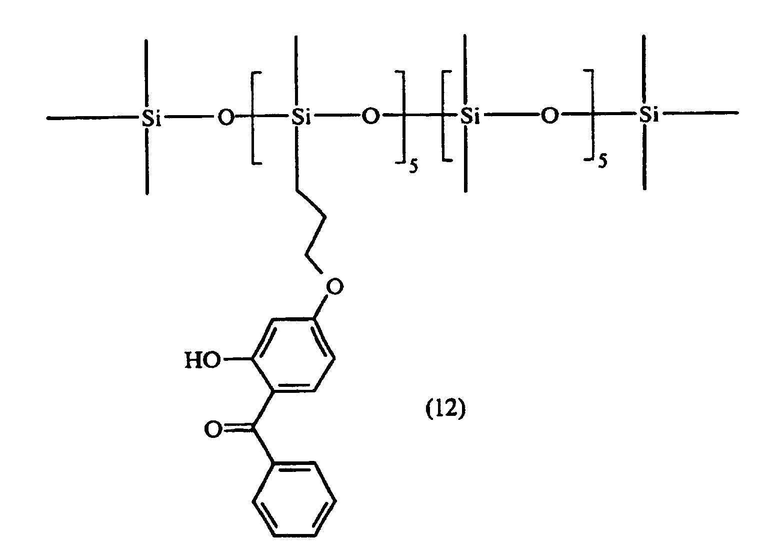

7. Composition selon la revendication 6, **caractérisée par le fait que** le dérivé de dibenzoylméthane est choisi parmi :
- le 2-méthyldibenzoylméthane
- le 4-méthyldibenzoylméthane
- le 4-isopropyldilbenzoylméthane
- le 4 tert.-butyldibenxoylméthane
- le 2,4-diméthyldibenzoylméthane
- le 2,5 diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

8. Composition selon la revendication 7, **caractérisée par le fait que** le dérivé de dibenzoylméthane est le 4-(tert-butyl) 4'-méthoxy dibenzoylméthane.

9. Composition selon la revendication 7, **caractérisée par le fait que** le dérivé de dibenzoylméthane est le 4-isopropyl-dibenzoylméthane.

10. Composition selon l'une quelconque des revendications 6 à 9, **caractérisée par le fait que** la teneur en dérivé(s) du dibenzoylméthane est comprise entre 0,01% et 10% en poids par rapport au poids total de la composition.

11. Composition selon la revendication 10, **caractérisée par le fait que** ladite teneur est comprise entre 0,3% et 5% en poids par rapport au poids total de la composition.

12. Composition selon la revendication 6 où le composé organosiloxanique est le composé de structure :

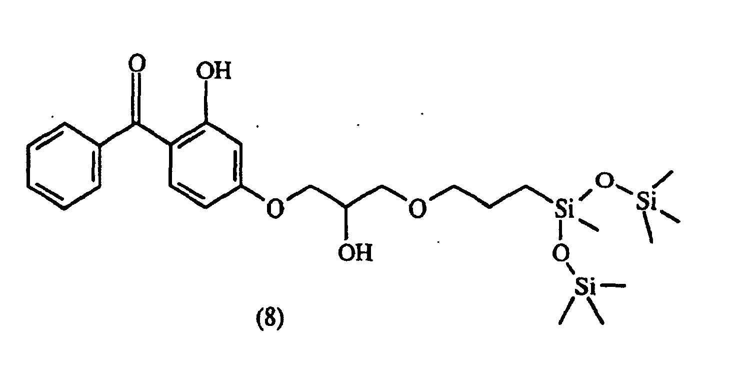

13. Composition selon l'une quelconque des revendications 6 à 12, **caractérisée par le fait que** la teneur en composé(s) organosiloxanique (s) est au moins égale à 0,5% en poids, par rapport au poids total de la composition.

14. Composition selon la revendication 13, **caractérisée par le fait que** ladite teneur va de 0,5% à 20% en poids, par rapport au poids total de la composition.

15. Composé choisi parmi les composés de formule suivantes :

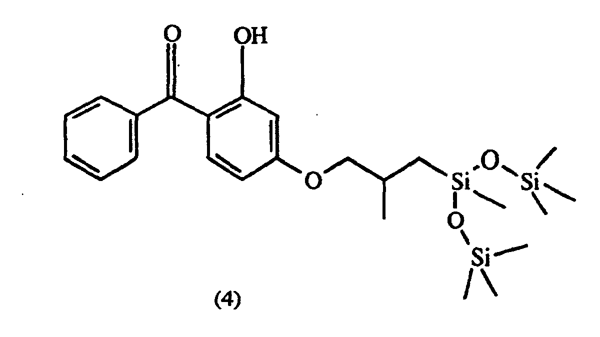

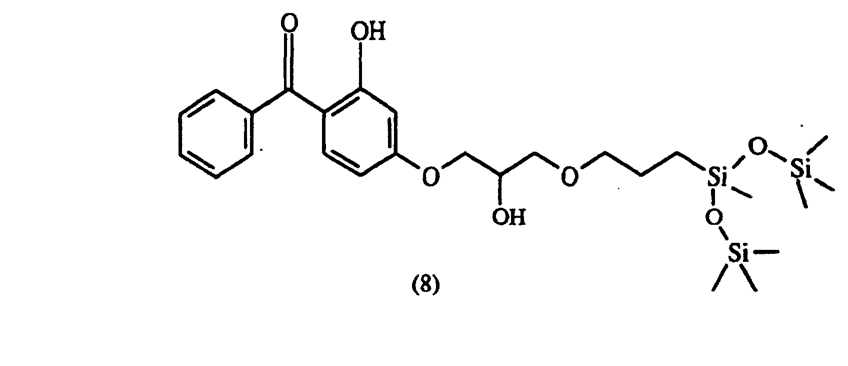

16. Composé de structure :

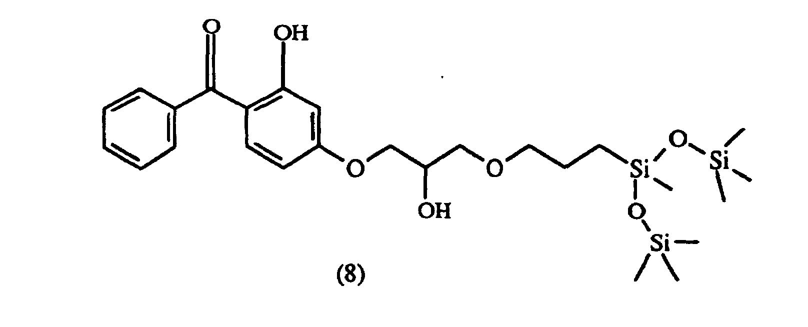

17. Utilisation d'un composé organosiloxanique à fonction 2-hydroxyphénone selon l'une des revendications 1 ou 6, dans la préparation d'une composition cosmétique ou dermatologique contenant au moins un dérivé du dibenzoylméthane, pour améliorer la stabilité dudit dérivé du dibenzoylméthane vis-à-vis du rayonnement UV.

## Claims

1. Process for improving the stability of at least one dibenzoylmethane derivative with respect to UV radiation, **characterized in that** it consists in combining an effective amount of an organosiloxane compound containing a 2-hydroxybenzophenone function with the said dibenzoylmethane derivative, chosen from the compounds of the following formulae:

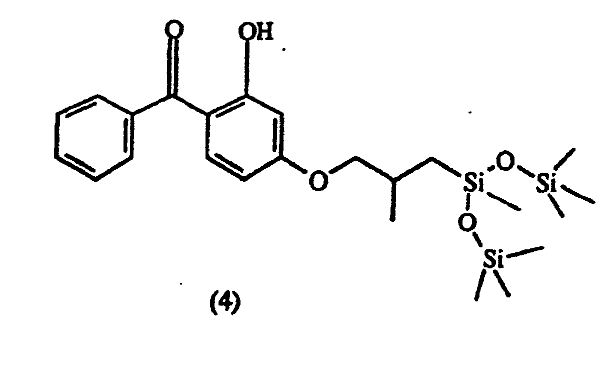

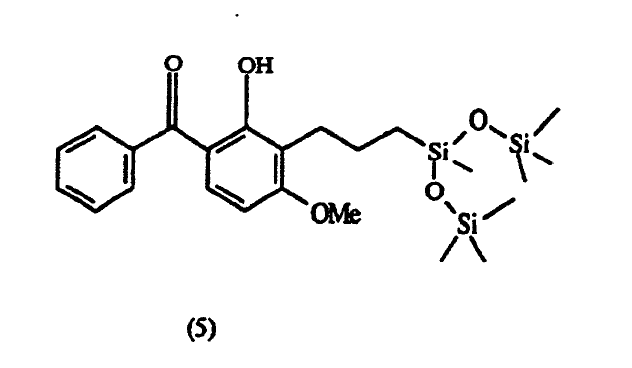

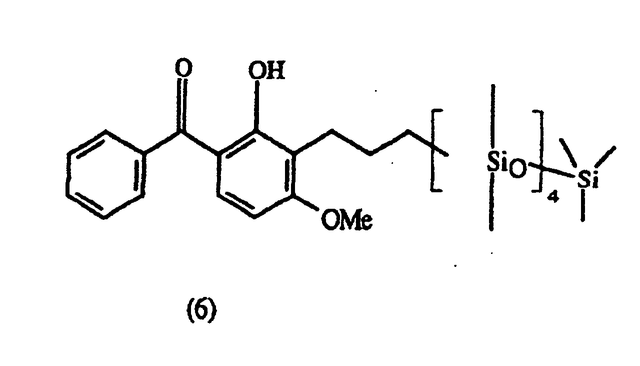

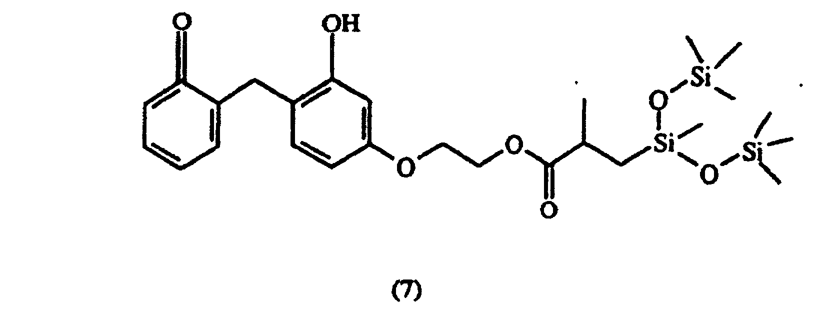

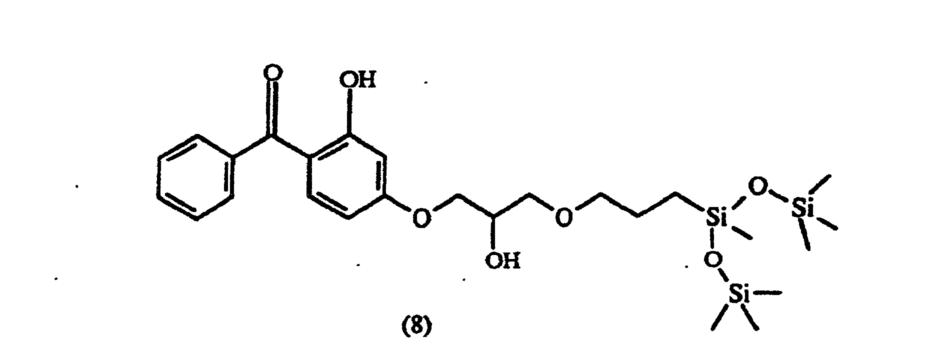

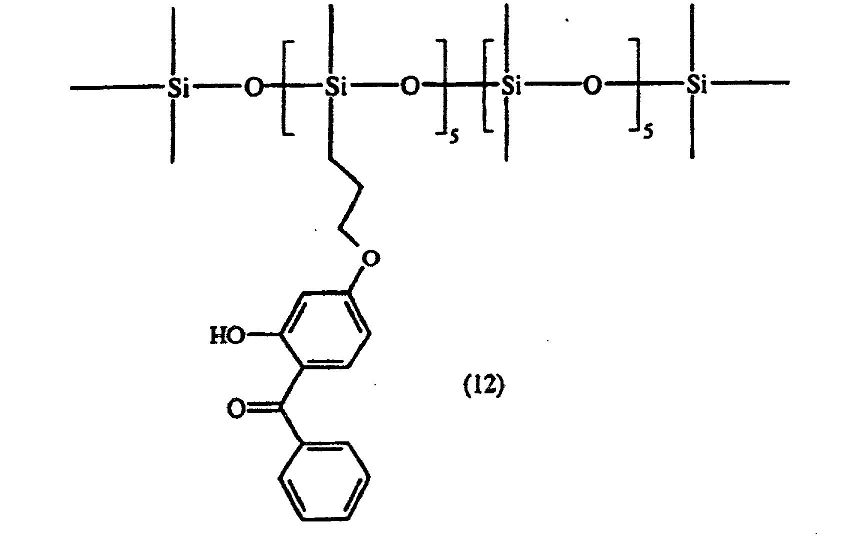

2. Process according to Claim 1, in which the organosiloxane compound is the compound of structure:

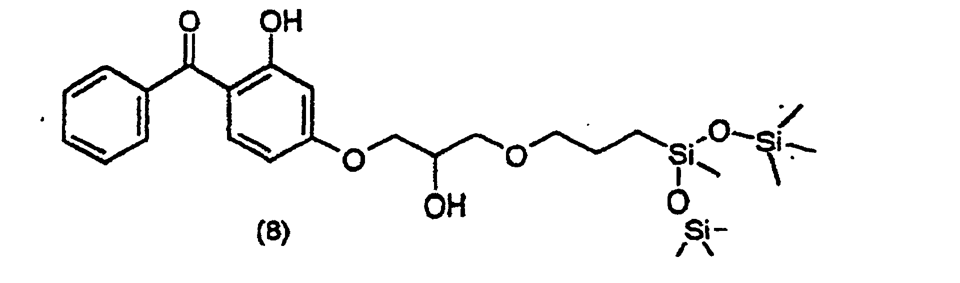

3. Process according to either of Claims 1 and 2, **characterized in that** the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

4. Process according to Claim 3, **characterized in that** the dibenzoylmethane derivative is 4-(tert-butyl)-4'-methoxydibenzoylmethane.

5. Process according to Claim 3, **characterized in that** the dibenzoylmethane derivative is 4-isopropyldibenzoylmethane.

6. Photostable screening cosmetic composition for the topical photoprotection of the skin and/or the hair against ultraviolet radiation, in particular solar radiation, of the type comprising, in a cosmetically acceptable support, at least one dibenzoylmethane derivative, **characterized in that** it also comprises an effective amount of at least one silane or organosiloxane compound containing a 2-hydroxybenzophenone function chosen from the compounds of the following formulae:

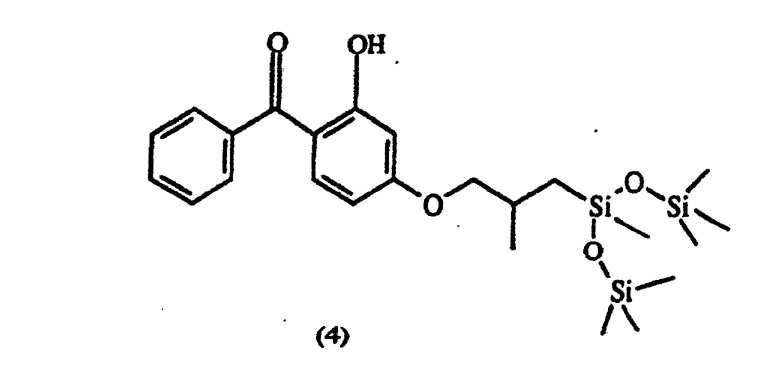

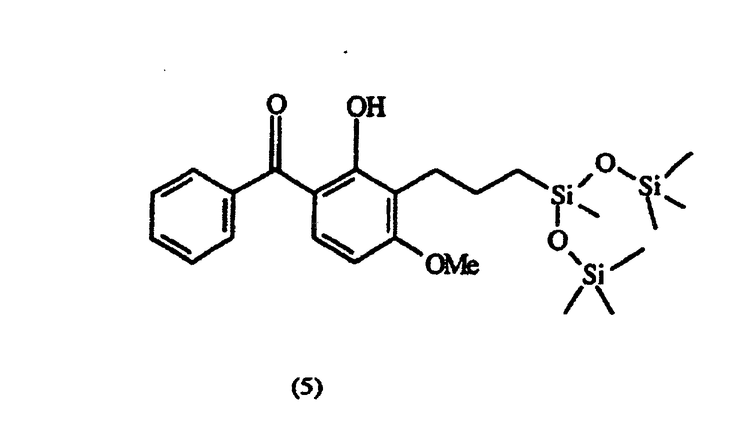

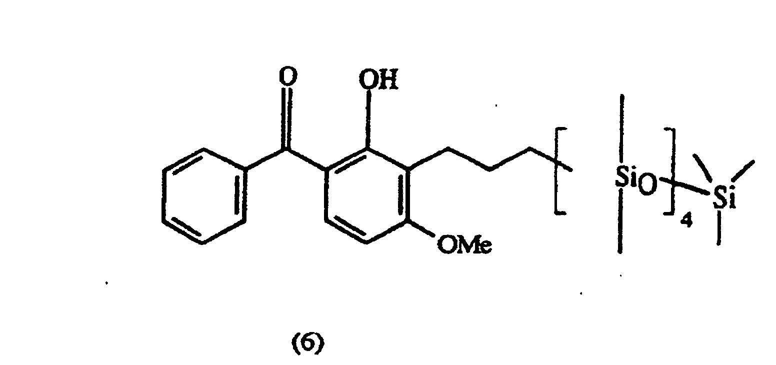

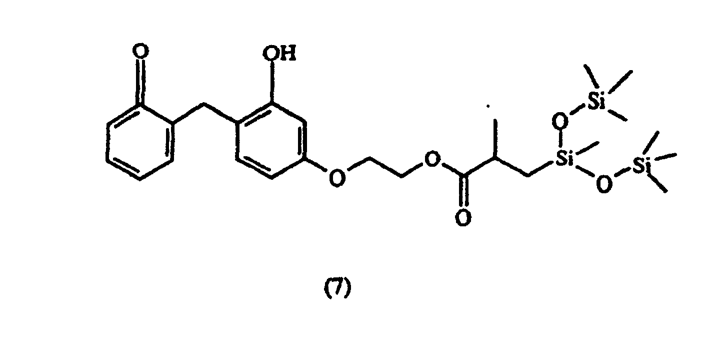

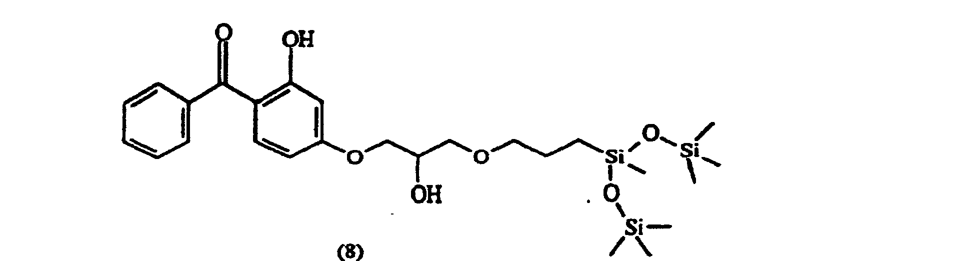

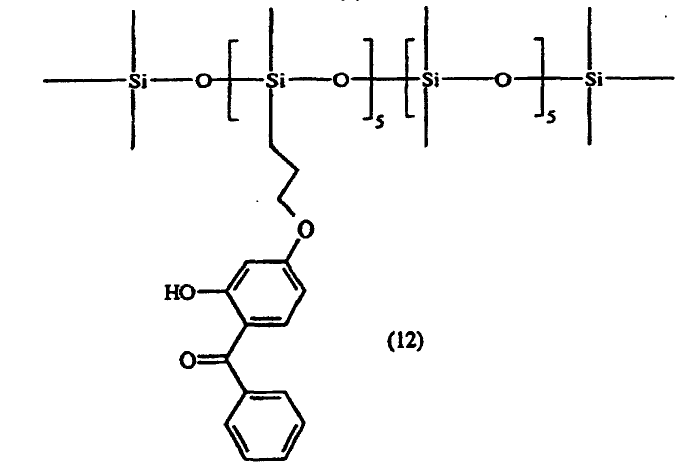

7. Composition according to Claim 6, **characterized in that** the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

8. Composition according to Claim 7, **characterized in that** the dibenzoylmethane derivative is 4-(tert-butyl)-4'-methoxydibenzoylmethane.

9. Composition according to Claim 7, **characterized in that** the dibenzoylmethane derivative is 4-isopropyldibenzoylmethane.

10. Composition according to any one of Claims 6 to 9, **characterized in that** the content of dibenzoylmethane derivative(s) is between 0.01% and 10% by weight relative to the total weight of the composition.

11. Composition according to Claim 10, **characterized in that** the said content is between 0.3% and 5% by weight relative to the total weight of the composition.

12. Composition according to Claim 6, in which the organosiloxane compound is the compound of structure:

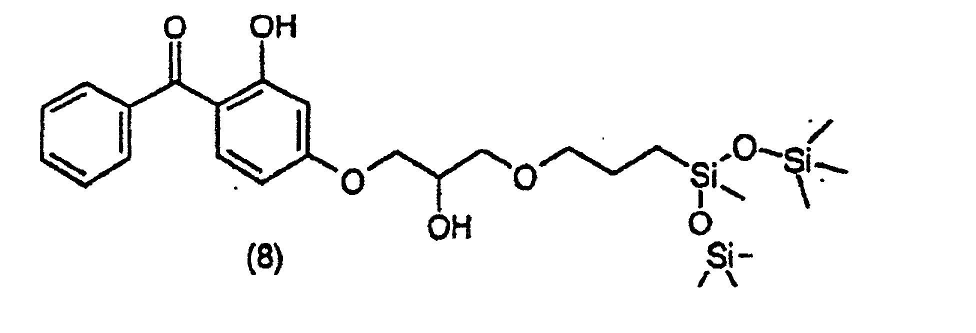

13. Composition according to any one of Claims 6 to 12, **characterized in that** the content or organosiloxane compound(s) is at least equal to 0.5% by weight relative to the total weight of the composition.

14. Composition according to Claim 13, **characterized in that** the said content ranges from 0.5% to 20% by weight relative to the total weight of the composition.

15. Compound chosen from the compounds of the following formulae:

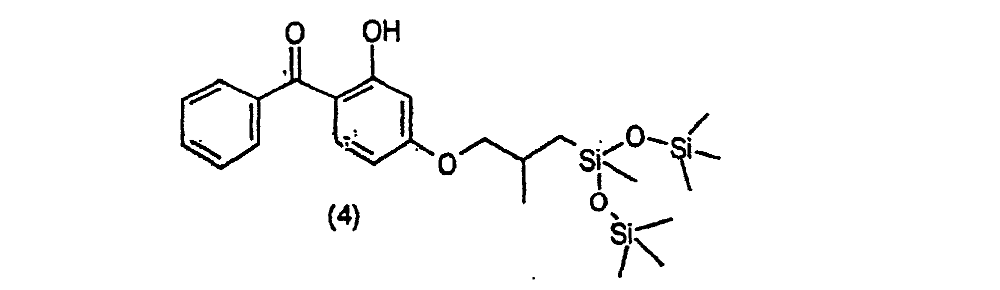

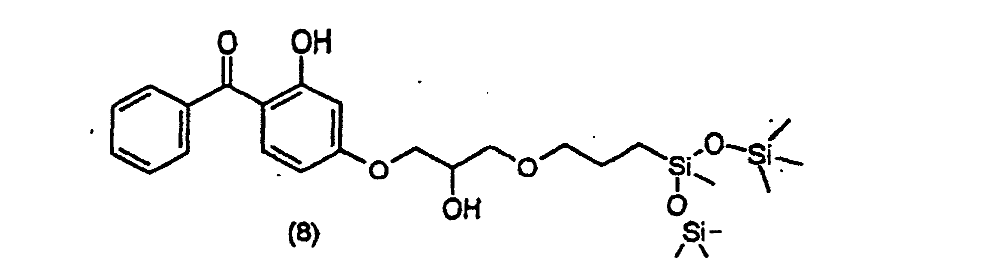

16. Compound of structure:

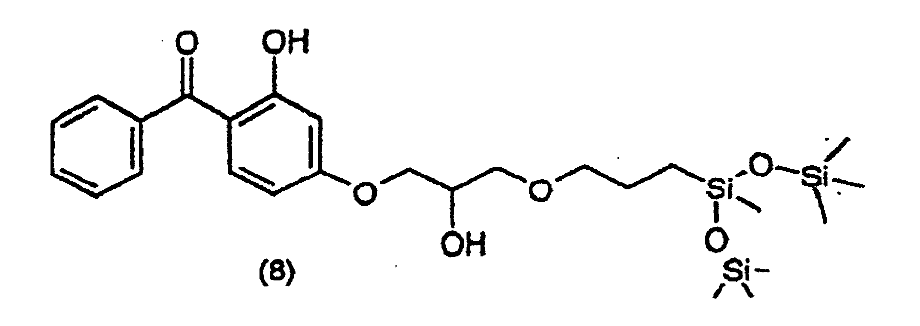

17. Use of an organosiloxane compound containing a 2-hydroxyphenone function, according to either of Claims 1 or 6, in the preparation of a cosmetic or dermatological composition containing at least one dibenzoylmethane derivative, to improve the stability of the said dibenzoylmethane derivative with respect to UV radiation.

## Patentansprüche

1. Verfahren zur Verbesserung der Stabilität mindestens eines Dibenzoylmethanderivats gegenüber UV-Strahlung, **dadurch gekennzeichnet, dass** es darin besteht, das Dibenzoylmethanderivat mit einer wirksamen Menge eines Organosiloxans mit 2-Hydroxybenzophenonfunktion zu kombinieren, das unter den Verbindungen der folgenden Formeln ausgewählt ist:

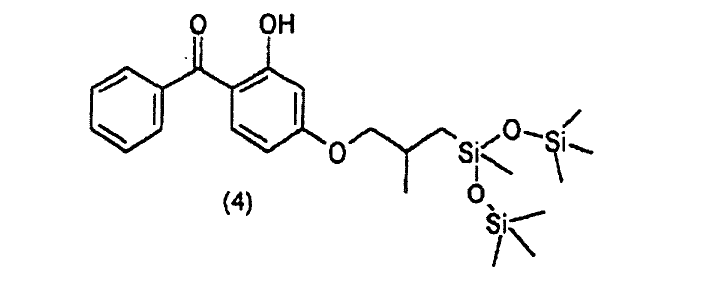

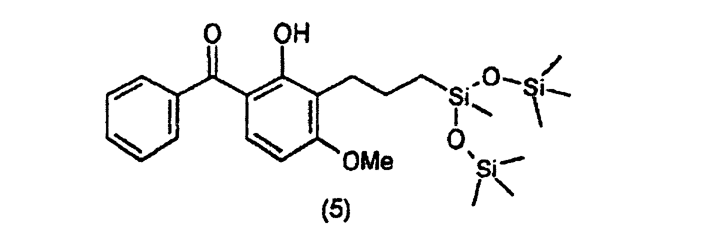

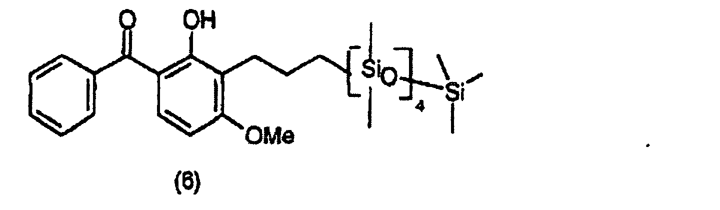

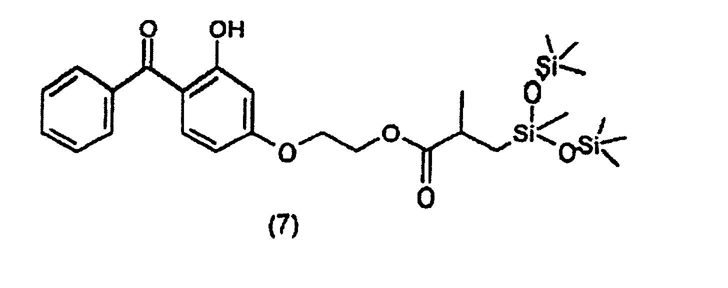

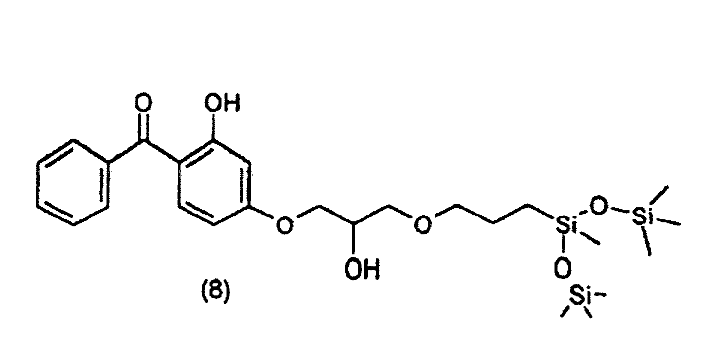

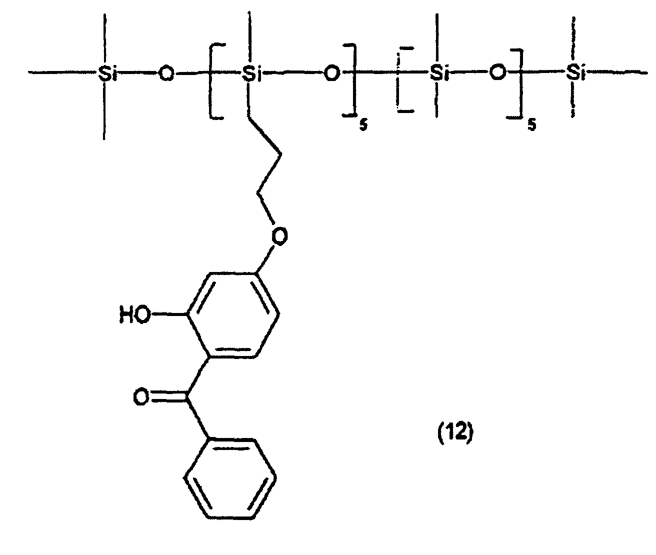

2. Verfahren nach Anspruch 1, wobei das Organosiloxan die Verbindung mit der folgenden Struktur ist:

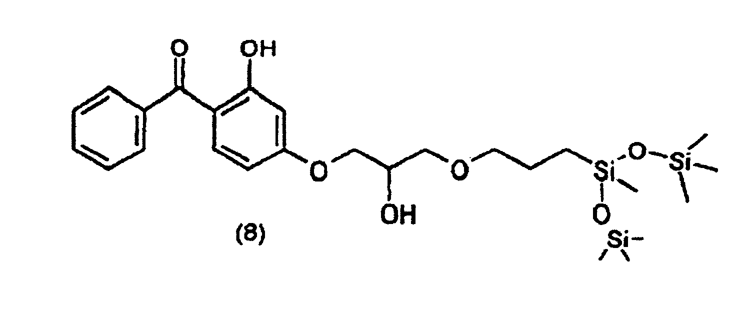

3. Verfahren nach einem der Ansprüche 1 oder 2, **dadurch gekennzeichnet, dass** das Dibenzoylmethanderivat ausgewählt ist unter:
- 2-Methyldibenzoylmethan
- 4-Methyldibenzoylmethan
- 4-Isopropyldibenzoylmethan
- 4-*t*-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-*t*-Butyl-4'-methoxydibenzoylmethan
- 2-Methyl-5-isopropyl-4'-methoxydibenzoylmethan
- 2-Methyl-5-*t*-butyl-4'-methoxydibenzoylmethan
- 2,4-Dimethyl-4'-methoxydibenzoylmethan
- 2,6-Dimethyl-4-*t*-butyl-4'-methoxydibenzoylmethan.

4. Verfahren nach Anspruch 3, **dadurch gekennzeichnet, dass** das Dibenzoylmethanderivat das 4-(*t*-Butyl)-4'-methoxydibenzoylmethan ist.

5. Verfahren nach Anspruch 3, **dadurch gekennzeichnet, dass** das Dibenzoylmethanderivat das 4-Isopropyldibenzoylmethan ist.

6. Photostabile filternde kosmetische Zusammensetzung zum Lichtschutz der Haut und/oder der Haare gegen UV-Strahlung und insbesondere gegen Sonnenlicht auf topischem Wege, die in einem kosmetisch akzeptablen Träger mindestens ein Dibenzoylmethanderivat enthält, **dadurch gekennzeichnet, dass** sie ferner in einer wirksamen Menge mindestens ein Organosiloxan mit 2-Hydroxybenzophenonfunktion enthält, das unter den Verbindungen der folgenden Formeln ausgewählt ist:

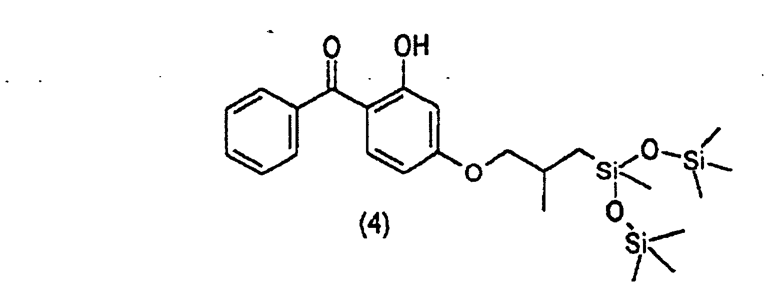

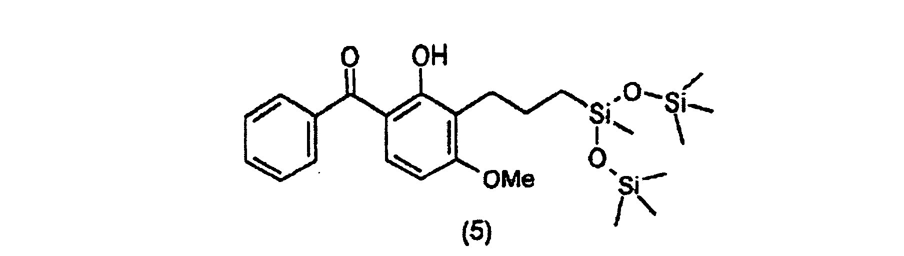

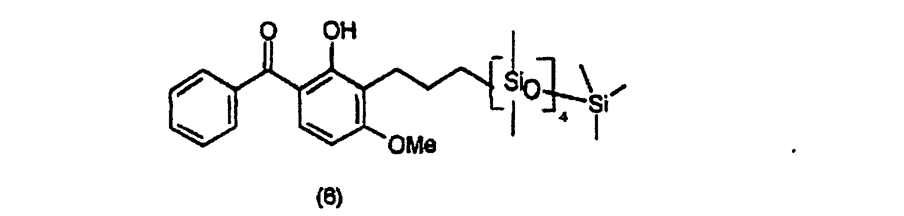

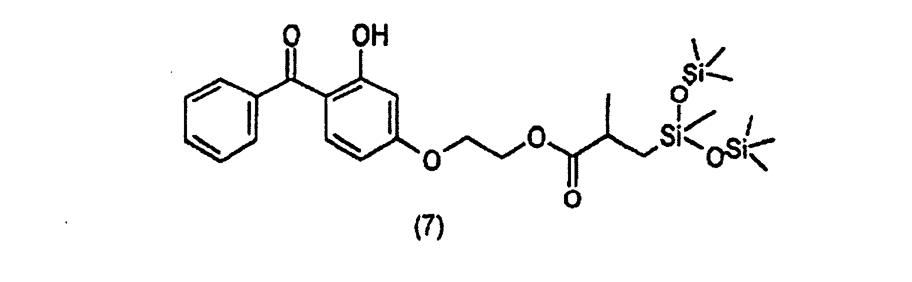

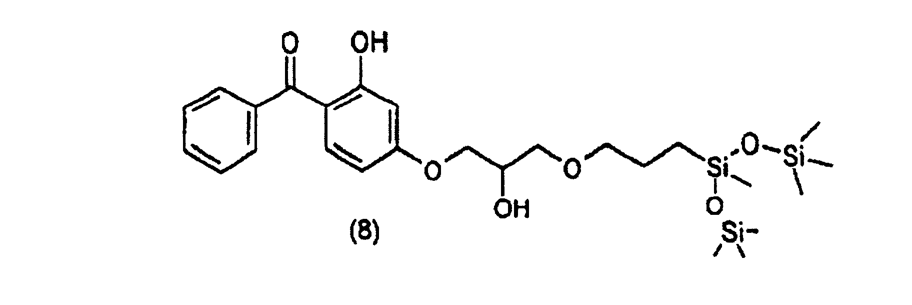

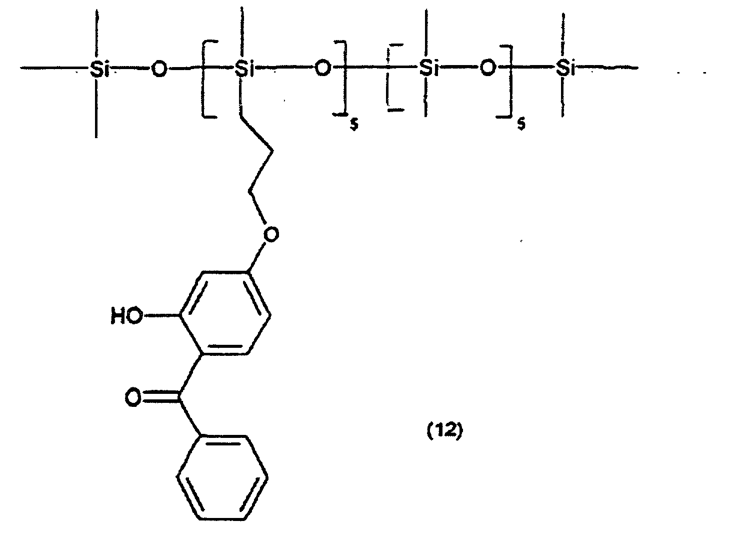

7. Zusammensetzung nach Anspruch 6, **dadurch gekennzeichnet, dass** das Dibenzoylmethanderivat ausgewählt ist unter:
- 2-Methyldibenzoylmethan
- 4-Methyldibenzoylmethan
- 4-Isopropyldibenzoylmethan
- 4-*t*-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-*t*-Butyl-4'-methoxydibenzoylmethan
- 2-Methyl-5-isopropyl-4'-methoxydibenzoylmethan
- 2-Methyl-5-*t*-butyl-4'-methoxydibenzoylmethan
- 2,4-Dimethyl-4'-methoxydibenzoylmethan
- 2,6-Dimethyl-4-*t*-butyl-4'-methoxydibenzoylmethan.

8. Zusammensetzung nach Anspruch 7, **dadurch gekennzeichnet, dass** das Dibenzoylmethanderivat das 4-(*t*-Butyl)-4'-methoxydibenzoyl-methan ist.

9. Zusammensetzung nach Anspruch 7, **dadurch gekennzeichnet, dass** das Dibenzoylmethanderivat das 4-Isopropyldibenzoylmethan ist.

10. Zusammensetzung nach einem der Ansprüche 6 bis 9, **dadurch gekennzeichnet, dass** der Gehalt des Dibenzoylmethanderivats oder der Dibenzoylmethanderivate im Bereich von 0,01 bis 10 Gew.-%, bezogen auf das Gesamtgewicht der Zusammensetzung, liegt.

11. Zusammensetzung nach Anspruch 10, **dadurch gekennzeichnet, dass** der Mengenanteil im Bereich von 0,3 bis 5 Gew.-%, bezogen auf das Gesamtgewicht der Zusammensetzung, liegt.

12. Zusammensetzung nach Anspruch 6, wobei das Organosiloxan die Verbindung der folgenden Struktur ist:

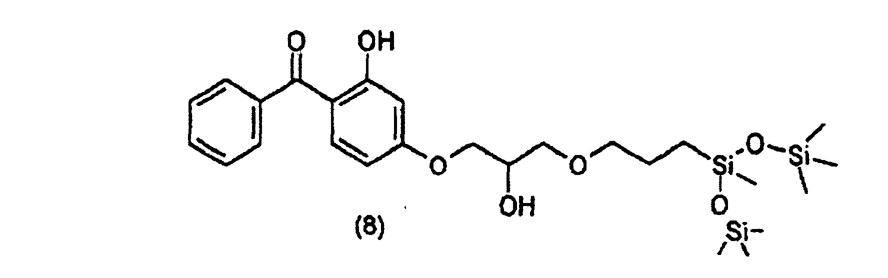

13. Zusammensetzung nach einem der Ansprüche 6 bis 12, **dadurch gekennzeichnet, dass** der Gehalt des Organosiloxans oder der Organosiloxane mindestens 0,5 Gew.-%, bezogen auf das Gesamtgewicht der Zusammensetzung, beträgt.

14. Zusammensetzung nach Anspruch 13, **dadurch gekennzeichnet, dass** der Mengenanteil im Bereich 0,5 bis 20 Gew.-%, bezogen auf das Gesamtgewicht der Zusammensetzung, liegt.

15. Verbindung, die unter den Verbindungen der folgenden Formeln ausgewählt ist:

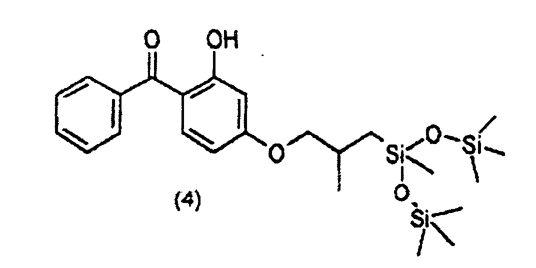

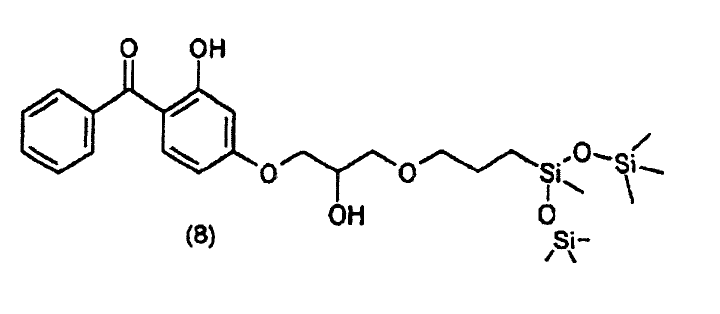

16. Verbindung der Struktur:

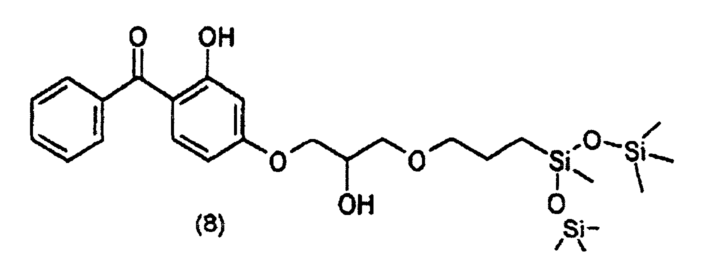

17. Verwendung eines Organosiloxans mit 2-Hydroxybenzophenonfunktion nach einem der Ansprüche 1 oder 6 für die Herstellung einer kosmetischen oder dermatologischen Zusammensetzung, die mindestens ein Dibenzoylmethanderivat enthält, zur Verbesserung der Stabilität des Dibenzoylmethanderivats gegenüber UV-Strahlung.
